# EUROPEAN PATENT APPLICATION

(11) **EP 2 551 674 A1**
(43) Date of publication of application: **30.01.2013**
(21) Application number: 11382255.5
(22) Date of filing: 26.07.2011
(51) Int. Cl.: G01N 33/68

(54) **MT1-MMP substrates as biomarkers of inflammatory angiogenesis**

(71) Applicant: Fundacion Centro Nacional De Investigaciones Cardiovasculares Carlos III (CINC), 28029 Madrid (ES); Institució Catalana De Recerca I Estudis Avançats (ICREA), 08010 Barcelona (ES); Fundació Privada Institut d'Investigació Oncològica de Vall d'Hebron, 08035 Barcelona (ES); Fundació Hospital Universitari Vall d' Hebron - Institut de Recerca, 08035 Barcelona (ES)
(72) Inventor: García Arroyo, Alicia, 28029 Madrid (ES); Koziol, Agnieszka, 28029 Madrid (ES); Canals, Francesc, 08035 Barcelona (ES); Colomé Calls, Núria, 08035 Barcelona (ES); Arribas, Joaquín, 08035 Barcelona (ES)
(74) Representative: Illescas, Manuel

(57) **Abstract**

**MT1-MMP substrates as biomarkers of inflammatory angiogenesis.** The present invention is mainly focused on searching and validating Membrane Type 1 Matrix Metalloprotease (MT1-MMP) substrates, in order to identify a specific group thereof which can be used as biomarker of inflammatory angiogenesis and, consequently, for the *in vitro* diagnosis or prognosis of disorders, having said etiology in common, for example psoriasis, rheumatoid arthritis and atherosclerosis.

## Description

### FIELD OF THE INVENTION

The present invention may be included in the field of the medicine in general, and, more particularly, in that area of the medicine which is focused on the study of aberrant inflammatory angiogenesis (the physiological process caused by inflammation involving the abnormal growth of new blood vessels from pre-existing vessels) and its implication in the development of chronic inflammatory disorders for example: psoriasis, rheumatoid arthritis and atherosclerosis.

The present invention is mainly directed to the search and validation of Membrane Type 1 Matrix Metalloprotease (MT1-MMP) substrates, in order to identify a specific group thereof which can be used as biomarker of inflammatory angiogenesis and, consequently, for the *in vitro* diagnosis or prognosis of disorders, having inflammatory angiogenesis as etiology in common, for example: psoriasis, rheumatoid arthritis and atherosclerosis.

### STATE OF THE ART

MT1-MMP is a key protease in angiogenesis. It can impact angiogenesis through the processing of matrix components such as fibrin, laminin, fibronectin and fibrillar collagens; or transmembrane molecules such as semaphorin 4D and endoglin. MT1-MMP is the main fibrinolysin and collagenase enzyme in endothelial cells. Therefore MT1-MMP is required for capillary sprouting and null mice display defective FGF-induced corneal angiogenesis and tissue defects related to reduced vascularization, such as impaired secondary ossification and lung expansion. Moreover said null mice display also impaired responses to angiogenic factors such as FGF and MCP-1/CCL2.

Angiogenesis requires the presence of invasive endothelial tip cells at the nascent sprout. Capillary sprouting is often coupled to inflammation, and inflammatory factors such as TNFα, SIP and bradykinin induce the *in vitro* formation of endothelial tip-like cells and the expression of the markers Jagged1 and VEGFR2, similarly to the VEGF/Notch pathways during vascular development. MT1-MMP expression has been observed in endothelial tip cells *in vitro* and mathematical models suggest that MT1-MMP contributes to endothelial tip cell guidance. Inflammatory mediators, such as nitric oxide (a downstream effector of bradykinin), prostaglandin E2 (PGE2) and the chemokine MCP-1/CCL2, increase MT1-MMP cell surface clustering and activity in human endothelial cells (ECs). All said factors require MT1-MMP for proper induction of capillary tube formation. MT1-MMP is therefore likely to be proteolytically active at the developing sprout. However, the activity of MT1-MMP in endothelial tip cells, in particular during inflammation, remains largely uncharacterized.

One of the most powerful ways to characterize the function of a protease is the identification of its full set of substrates (known as the protease degradome) (*Doucet A et al 2008, Overall CM et al 2006).* Several shot-gun proteomics approaches have been developed and used for degradome identification, and each of them has advantages and disadvantages. Although the recently developed technique SILAC (stable isotope labeling of amino acids in cell culture) has numerous advantages (*Ong SE et al 2002),* MT1-MMP substrates have not been previously characterized by SILAC comparison of wildtype (WT) and MT1-MMP deficient mouse primary cells.

Therefore, knowing that MT1-MMP is as a critical factor in the interplay between inflammation and angiogenesis (during capillary sprouting, specialized endothelial tip cells are formed and express the protease MT1-MMP), that MT1-MMP is involved in tip cell invasion of the surrounding tissue, and that, as cited above, the most powerful way to characterize the function of a protease is to identify its full set of substrates (degradome), the present invention is focused on searching and validating Membrane Type 1 Matrix Metalloprotease (MT1-MMP) substrates, in order to identify specific combinations thereof which can be efficiently used as biomarkers of inflammatory angiogenesis and, consequently, for the *in vitro* diagnosis or prognosis of disorders, having said etiology in common, for example: psoriasis, rheumatoid arthritis and atherosclerosis.

### DESCRIPTION OF THE INVENTION

As explained above, the present invention is mainly focused on the identification and validation of MT1-MMP substrates, preferably specific groups or combinations thereof, to be used as biomarkers of inflammatory angiogenesis and, consequently, for the *in vitro* diagnosis or prognosis of disorders or diseases sharing said etiology, for example psoriasis, rheumatoid arthritis and atherosclerosis. Persistent and aberrant angiogenesis is a hallmark of chronic inflammatory disorders such as the aforesaid diseases psoriasis, rheumatoid arthritis and atherosclerosis. Therefore angiogenesis has been proposed as a therapeutic target for these conditions. Profiling of MT1-MMP processed substrates, preferably in plasma, can therefore be used as a surrogate biomarker of angiogenic activity in chronic inflammatory diseases, potentially identifying patients who might benefit of anti-angiogenic therapies.

In order to achieve said objective, SILAC technique was used, in the present invention, to identify MT1-MMP substrates in TNFα-activated endothelial cells. Moreover, selected substrates were subsequently validated in 2D and 3D endothelial cell models, and also in mouse retinas *ex vivo,* highlighting the relevance of MT1-MMP-mediated protein processing in inflammatory angiogenesis.

SILAC was elected as proteomic strategy to characterize the group of MT1-MMP substrates (degradome) in primary endothelial cells activated by the inflammatory cytokine TNFα, because is one of the most powerful shot-gun quantitative proteomic approaches used for comparison of proteomes in different cellular conditions. Nevertheless, its use is restricted in low-proliferative and terminally-differentiated primary cells because of its requirement for several cell doublings (i.e. divisions or multiplication by 2). However, this drawback was circumvented in the present invention by means of the use of immortalized primary mouse lung endothelial cells. These cells maintain their functional properties but, at the same time, activate signaling pathways that increase proliferation and MT1-MMP activity, thus providing optimal conditions for analyzing MT1-MMP proteolytic events. Using this approach, it was possible to perform SILAC for the first time, in the present invention, in endothelial cells obtained from genetically-modified mice, characterizing the MT1-MMP degradome in endothelial tip cells in the setting of inflammation.

Thus, following this approach, several proteins were initially identified by SILAC in the supernatants of wild-type (WT) (using heavy "H" labeling conditions by culturing the cells in the presence of forms of the amino acids (isotopes) with higher molecular mass), and MT1-MMP null (KO) immortalized endothelial cells (using light "L" labeling conditions by culturing the cells in the presence of forms of the amino acids (isotopes) with lower molecular mass), defining their relative H/L ratios based on isotope labeling, and selecting those proteins having an H/L ratio >1.5 (see **Examples 2** and **3).** The H/L ratio for a protein is calculated estimating the average of the H/L ratios for the peptides identified for each protein. Regarding the interpretation of the ratio, a transmembrane protein shed by the protease will yield an H/L ratio >1 meaning more abundance of the soluble form in the presence of the protease (wild-type or 'heavy' condition). However, a secreted protein might yield an H/L ratio >1 or <1 depending on the pattern of processing by the protease i.e. either released from the matrix or processed into small undetectable fragments (see **Examples 2** and **3**). To increase potential selectivity of identified substrates a cut-off H/L ratio=1.5 was chosen based on the ratio of the first previously identified MT1-MMP substrate present in the list with a ratio >1, i.e. collagen III. Therefore, as explained in the examples, a specific group of MT1-MMP substrates, relevant to different biological processes related with angiogenesis, were identified and validated departing from said initially identified proteins (see **Table 1**), providing an integrated view of how MT1-MMP contributes to capillary sprouting, by means of the combinatorial cleavage of defined sets or groups of substrates.

**Table 1**

| **Symbol** | **Protein name** | **Protein description** |
|---|---|---|
| ITPR1 | Inositol 1,4,5-trisphosphate receptor type 1 | Calcium signaling pathway |
| TSP2 | Thrombospondin-2 | Cell adhesion, ECM-receptor interaction |
| MMP3 | Stromelysin-1 | Proteolysis |
| LYOX | Protein-lysine 6-oxidase | Vasculature development, extracellular matrix organization |
| PCOCI | Procollagen C-endopeptidase enhancer 1 | Proteolysis |
| TRPM2 | Transient receptor potential cation channel subfamily M member 2 | Ion transport |
| EGFR | Epidermal growth factor receptor | Proliferation, protein interaction |
| SODE | Extracellular superoxide dismutase [Cu-Zn] | Oxygen metabolism |
| PTX3 | Pentraxin-related protein PTX3 | Membrane organization, endocytosis |
| PR2C2 | Prolactin-2C2 | Vascular development, cell migration |
| IC1 | Plasma protease C 1 inhibitor | Immune response |
| CNTN1 | Contactin-1 | Cell adhesion |
| STC1 | Stanniocalcin-1 | Cellular ion homeostasis |
| SFRP1 | Secreted frizzled-related protein 1 | Development, differentiation |
| TIMP1 | Metalloproteinase inhibitor 1 | Enzyme inhibitor activity |
| SEM3C | Semaphorin-3C | Vasculature development, axon guidance |
| CO6A1 | Collagen alpha-1(VI) chain | Cell adhesion |
| FBN1 | Fibrillin-1 | Metal ion binding |
| SLIT3 | Slit homolog 3 protein | Axon guidance |
| LIF | Leukemia inhibitory factor | Receptor interaction, Jak-STAT signaling pathway |
| CERU | Ceruloplasmin | Metal ion binding |
| NID1 | Nidogen-1 | Cell adhesion, binding |
| NGAL | Neutrophil gelatinase-associated lipocain | Response to virus |
| MMP19 | Matrix metalloproteinase-19 | Vascular development, differentiation |
| CSF1 | Macrophage colony-stimulating factor 1 | Cytokine-cytokine receptor interaction |
| GELS | Gelsolin | Cytoskeleton organization |
| TENA | Tenascin | Focal adhesion, ECM-receptor interaction |
| FBLN2 | Fibulin-2 | Cell adhesion, matrix binding |
| CO3 | Complement C3 | Immune response, ECM-receptor interaction |
| CCL2 | C-C motif chemokine 2 | Proliferation. Immune response, chemotaxis |
| TIMP3 | Metalloproteinase inhibitor 3 | Enzyme inhibitory activity |
| SLIT2 | Slit homolog 2 protein | Axon guidance, cell motion |
| TSP1 | Thrombospondin-1 | Migration, cell adhesion, immune response |
| CYR61 | Protein CYR61 | Vasculature development, adhesion, proliferation |
| CO3A1 | Collagen alpha-1(III) chain | Vasculature development |
| TIMP2 | Metalloproteinase inhibitor 2 | Enzyme inhibitory activity |
| KLRA4 | Killer cell lectin-like receptor 4 | Cell adhesion, NK mediated cytotoxicity |
| KPBB | Phosphorylase b kinase regulatory subunit beta | Calcium signaling pathway |
| SNED1 | Sushi, nidogen and EGF-like domain-containing protein 1 | Cell adhesion |
| CPXM1 | Probable carboxypeptidase X1 | Adhesion, proteolysis |
| IGS10 | Immunoglobulin superfamily member 10 | Receptor signaling |
| AEBP1 | Adipocyte enhancer-binding protein 1 | Regulation of transcription, cell adhesion |
| LOXL3 | Lysyl oxidase homolog 3 | Oxidoreductase activity |
| GDN | Glia-derived nexin | Enzyme inhibitory activity, developmental protein |

| | | |
|---|---|---|
| *List of MT1-MMP Substrates (H*/*L*>*1.5) with their generic assigned function.* | | |

Moreover, an additional step was carried out in the present invention with the aim of validating the most valuable MT1-MMP substrates, or combinations thereof, which could be efficiently used as biomarker of inflammatory angiogenesis and, consequently, for the *in vitro* diagnosis or prognosis of disorders caused by said mechanism.

All the MT1-MMP substrates comprised in **Table 1** can be independently used as biomarkers of inflammatory angiogenesis because they are independently related with different physiological processes involved in angiogenesis. However, the system biology analysis of the MT1-MMP endothelial degradome, carried out in the present invention, preferably suggests a modular organization of MT1-MMP substrate processing in a combinatorial manner. Therefore, MT1-MMP substrates were grouped into functional modules linked to biological processes related to angiogenesis. There is also a hierarchical organization of these substrates since some of them are present in several functional modules. The common component of the modules related to angiogenesis is TSP1, identifying this multi-domain matrix glycoprotein as a master regulator of biological processes including adhesion, motility, chemotaxis and morphogenesis, and potentially explaining the tight regulation and coordinated processing between MT1-MMP and ADAMTS1. The implication of these findings is that the outcome of angiogenesis will depend on combined rather than individual processing of substrates, whose fragments might act in a competitive, synergistic or sequential manner to fine-tune the angiogenic response. In this regard, several of the identified substrates share structural domains and are therefore able to interact with similar matrix proteins or cell receptors.

The present invention was initially focused on the validation of the MT1-MMP processing of these proteins: TSP1, NID1 and/or CYR61, which are in the first-line of the combinatorial program.

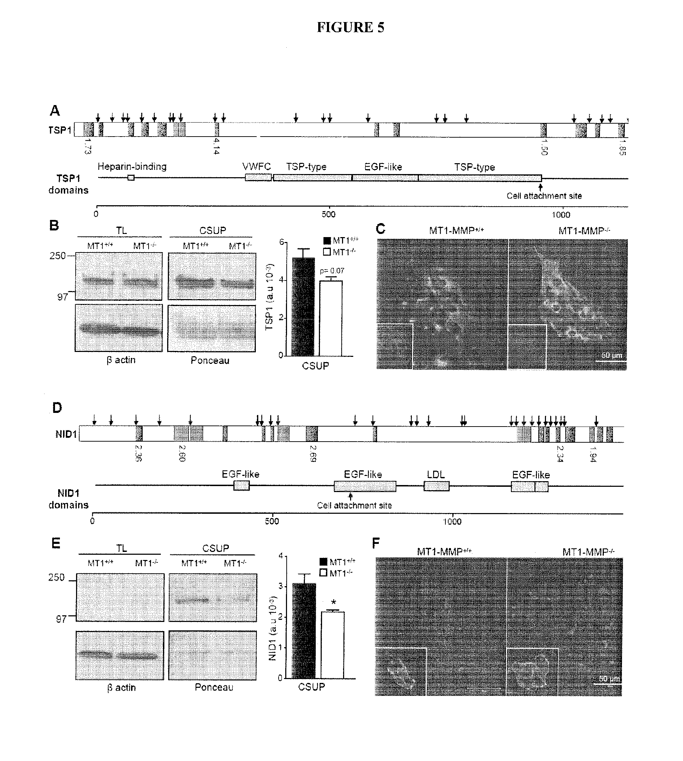

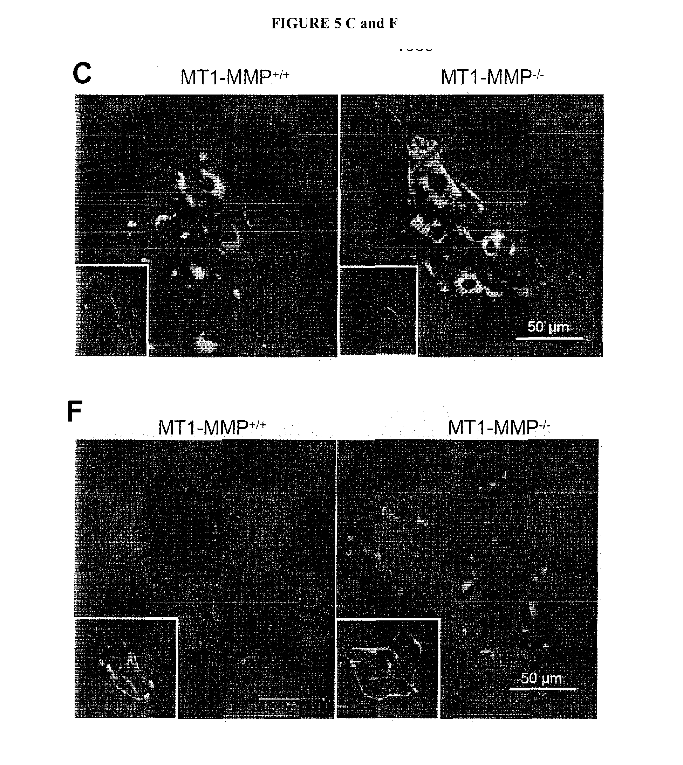

TSP1 can be cleaved by ADAMTS1, releasing a soluble C-terminal monomer and leaving a matrix-bound N-terminal trimeric fragment. During inflammatory angiogenesis, MT1-MMP further modulates the angiogenic properties of TSP1 either by cooperating with ADAMTS1 or by acting at a different cleavage site, affecting interaction with receptors such as integrin αvβ3, CD36 or CD47. Accumulation of TSP1 clusters in TNFα-stimulated MT1-MMP null lung endothelial cells (MLEC) indicates that MT1-MMP is required for TSP1 processing events that are not compensated for ADAMTS1. Increased pericellular TSP1 deposits might contribute to the reduced angiogenesis reported in MT1-MMP null cells. Thus, levels of soluble TSP1 were higher in culture supernatants of WT MLEC, indicating release of TSP1 from the matrix by MT1-MMP (**Figure 5B**). Accordingly, MT1-MMP-deficient MLEC accumulated larger TSP1 pericellular clusters compared with WT MLEC (**Figure 5C**). Therefore, it can be affirmed that the level of soluble TSP1 is higher in subjects suffering from inflammatory angiogenesis wherein MT1-MMP is activated (MT1^{+/+}), as compared with the corresponding levels of said MT1-MMP substrate in subjects not suffering from inflammatory angiogenesis wherein MT1-MMP is not activated (MT1^{-/-}) (see **Figure 5B** and **Example 6**). When MT1-MMP is activated (MT1^{+/+}), it digests TSP1 substrate attached to the extracellular matrix (ECM), increasing the level of TSP1 in soluble form.

NID1 contributes to basement membrane stability, and therefore the increased NID1 levels observed in MT1-MMP null endothelial cells might confer basement membrane-like 'quiescence' signals, thus hindering the angiogenic response by impeding endothelial cell engagement in active sprouting. Although NID1 was undetectable in total lysates, levels of soluble NID1 were significantly increased in culture supernatants from TNFα-stimulated WT pMLEC, suggesting MT1-MMP-dependent release of NID1 from the basement membrane (Figure 5E). Consistent with cleavage of NID1 at the N- or C-terminus, the molecular weight of NID 1 in supernatants from WT MLEC was slightly smaller (**Figure 5E**). Accordingly, MT1-MMP-deficient MLEC, but not their WT counterparts, accumulated NID1 at clusters in contact with the matrix (**Figure 5F**). Therefore, it can be affirmed that the level of soluble NID1 is significantly higher in subjects suffering from inflammatory angiogenesis wherein MT1-MMP is activated (MT1^{+/+}), as compared with the corresponding levels of said MT1-MMP substrate in subjects not suffering from inflammatory angiogenesis wherein MT1-MMP is not activated (MT1^{-/-}) (see **Figure 5E** and **Example 6**). When MT1-MMP is activated (MT1^{+/+}), it digests NID1 substrate attached to the extracellular matrix (ECM), increasing the level of NID1 in soluble form.

Impaired processing of TSP1 and NID1 was confirmed in TNFα-activated MT1-MMP-deficient MLEC, with decreased levels of their soluble forms accompanied by pericellular accumulation (see **Example 6**).

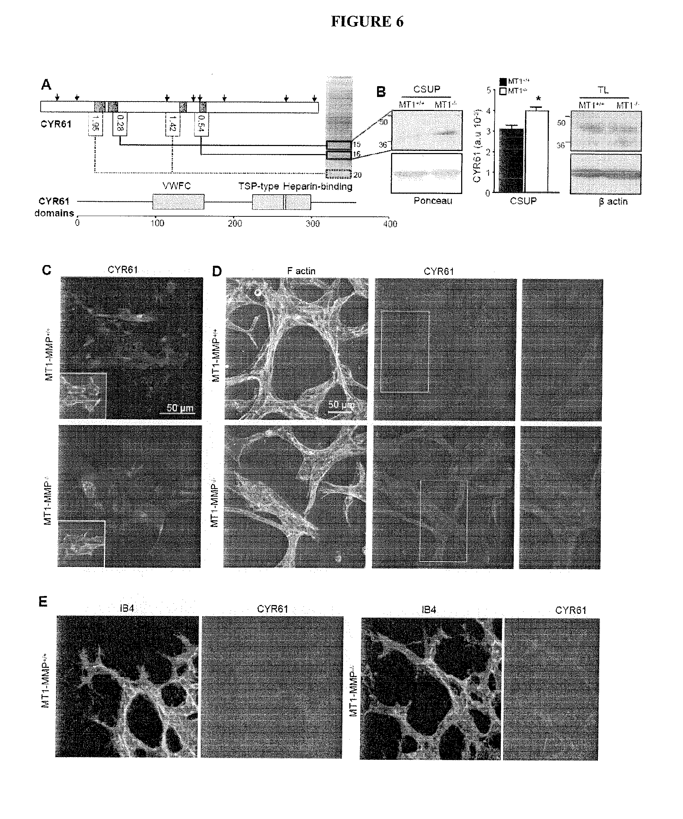

CYR61 (also known as CCN1), is an exclusive substrate of MT1-MMP within the metalloproteinase family. CYR61 levels in total lysates were unaffected by MT1-MMP deficiency, but, in contrast to TSP1 and NID1, levels of soluble CYR61 in supernatants of TNFα-activated WT MLEC were significantly lower than in supernatants of similarly treated null cells (**Figure 6B**). Therefore, it can be affirmed that the level of soluble CYR61 is significantly lower in subjects suffering from inflammatory angiogenesis wherein MT1-MMP is activated (MT1^{+/+}), as compared with the corresponding levels of said MT1-MMP substrate in subjects not suffering from inflammatory angiogenesis wherein MT1-MMP is not activated (MT1^{-/-}) (see **Example 7**).

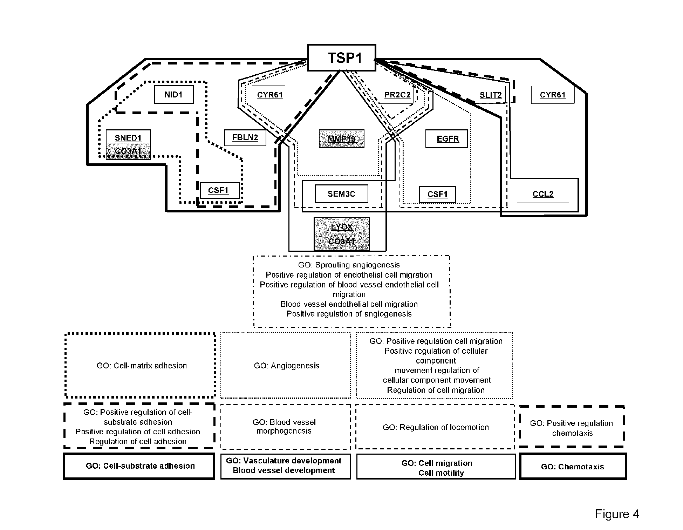

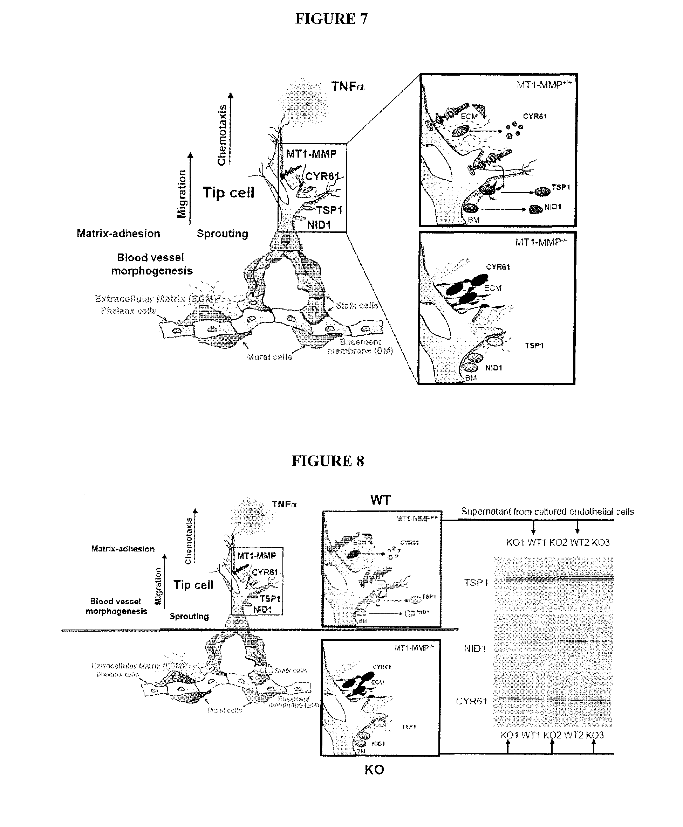

TSP1, NID1 or CYR61 are above discussed as individual events demonstrating that they can be individually used (or even in couples: TSP1 and NID1, TSP1 and CYR61, or NID1 and CYR61) as biomarkers of inflammatory angiogenesis because, as shown in **Figure 4** and **5****,** they are independently related with different physiological processes involved in angiogenesis. However, although the above cited proteins may be efficiently used as independent biomarkers of inflammatory angiogenesis, it is important to emphasize that the best results were obtained when at least the whole group of the three substrates (TSP1, NID1 and CYR61) was used (see **Figure 8****, Example 5** and **Example 8**). The functional interaction between the substrates TSP1, NID1 and CYR61 achieves a combined technical effect which is greater than the sum of the technical effects of the individual substrates since: 1) each of these proteins displays a different pattern of processing by MT1-MMP and then their soluble levels can be higher or lower depending on the type of cleavage (in the examples two of them TSP1 and NID1 display higher, and one of them CYR61 lower, levels of soluble forms in the presence of MT1-MMP; 2) combined quantifying would increase selectivity for MT1-MMP processing since some of these substrates can also be processed by other proteases in other contexts; and 3) current approaches to biomarker identification and personalized medicine requires a profiling of several proteins/genes to avoid random variability of a single biomarker in the general population so the combination will have more statistical power to avoid outliers. Consequently, by means of the combination of the three substrates (TSP1, NID1 and CYR61) it is possible to successfully foresee inflammatory angiogenesis because all the most important physiological processes which play a fundamental role during angiogenesis (cell-substrate adhesion, cell motility, cell migration, chemotaxis, vasculature and blood vessel development) can be identified, providing a stronger biomarker as compared with the individual use of said proteins. Moreover, MT1-MMP cleavage of TSP1, NID1 and CYR61 occurs within the same time-frame, thus highlighting the variety of processes impacted by the MT1-MMP proteolytic program during angiogenesis.

Moreover, the use of TSP1, NID1 and CYR61 can be combined with at least one of the following substrates: ITPR1, TSP2, MMP3, LYOX, PCOCI, TRPM2, EGFR, SODE, PTX3, PR2C2, IC1, CNTN1, STC1, SFRP1, TIMP1, SEM3C, CO6A1, FBN1, SLIT3, LIF, CERU, NGAL, MMP19, CSF1, GELS, TENA, FBLN2, CO3, CCL2, TIMP3, SLIT2, CO3A1, TIMP2, KLRA4, KPBB, SNED1, CPXM1, IGS10, AEBP1, LOXL3 or GDN, preferably with at least one of the following substrates: EGFR, PR2C2, SEM3C, CSF1, FBLN2, SLIT2, SNED1 or CCL2, all of them also having a clear relationship with physiological processes directly related with inflammatory angiogenesis (see **Table 1** and **Figure 4**), in order to improve the strength of the biomarker.

Consequently, in a preferred embodiment of the invention, the biomarker is comprised by one of the following group of substrates: TSP1, NID1, CYR61 and EGFR; TSP1, NIDI, CYR61 and PR2C2; TSP1, NID1, CYR61 and SEM3C; TSP1, NID1, CYR61 and CSF 1; TSP 1, NID1, CYR61 and FBLN2; TSP 1, NID1, CYR61 and SLIT2; TSP 1, NID1, CYR61 and SNED1; or TSP1, NID1, CYR61 and CCL2.

Therefore, the first embodiment of the present invention refers to the use of at least one of the MT1-MMP substrates comprised in **Table 1,** preferably the use of at least the following group of MT1-MMP substrates: TSP1, NID1 and CYR61, as biomarkers of inflammatory angiogenesis. In a preferred embodiment of the invention the use of the MT1-MMP substrates: TSP1, NID1 and CYR61, is combined with at least one of the following MT1-MMP substrates: EGFR, PR2C2, SEM3C, CSF1, FBLN2, SLIT2, SNED1 or CCL2. Moreover, the present invention also comprises the use of the following group of MT1-MMP substrates: TSP1, NID1, CYR61, EGFR, PR2C2, SEM3C, CSF1, FBLN2, SLIT2, SNED1 and CCL2 as biomarker of inflammatory angiogenesis.

For the purpose of the present invention the term "biomarker" refers to a molecule, mainly a MT1-MMP substrate or, more preferably, a group of MT1-MMP substrates, used as an indicator of a biological state, particularly inflammatory angiogenesis. Moreover the expression "inflammatory angiogenesis" refers to the process caused by inflammatory factors, for example TNF-α, involving the abnormal growth of new blood vessel from pre-existing vessel.

The second embodiment of the present invention is directed to the use of at least one of the MT1-MMP substrates listed in **Table 1,** preferably the use of the following group of MT1-MMP substrates: TSP1, NID1 and CYR61, as biomarkers for the *in vitro* diagnosis or prognosis of disorders caused by inflammatory angiogenesis. In a preferred embodiment of the invention the use of the MT1-MMP substrates: TSP1, NID1 and CYR61, is combined with at least one of the following MT1-MMP substrates: EGFR, PR2C2, SEM3C, CSF1, FBLN2, SLIT2, SNED1 or CCL2. Moreover, the present invention also comprises the use of the following group of MT1-MMP substrates: TSP1, NID1, CYR61, EGFR, PR2C2, SEM3C, CSF1, FBLN2, SLIT2, SNED1 and CCL2 as biomarker for the *in vitro* diagnosis or prognosis of disorders caused by inflammatory angiogenesis, for example: psoriasis, rheumatoid arthritis or atherosclerosis.

For the purpose of the present invention the wording "disorders caused by inflammatory angiogenesis" refers to all those diseases having inflammatory angiogenesis etiology, for example (non-exhaustive list): psoriasis, rheumatoid arthritis or atherosclerosis.

The third embodiment of the present invention refers to an *in vitro* method for the diagnosis or prognosis of inflammatory angiogenesis which comprises assessing the soluble level of at least one of the MT1-MMP substrates comprised in **Table 1,** preferably of at least the following group of MT1-MMP substrates: TSP1, NID1 and CYR61, in samples isolated from the subject under investigation. In a preferred embodiment of the invention the method is based on assessing the soluble levels of the MT1-MMP substrates: TSP1, NID1 and CYR61, in combination with at least one of the following MT1-MMP substrates: EGFR, PR2C2, SEM3C, CSF1, FBLN2, SLIT2, SNED1 or CCL2. Moreover, the method of the invention also comprises assessing the soluble level of the following group of MT1-MMP substrates: TSP1, NID1, CYR61, EGFR, PR2C2, SEM3C, CSF1, FBLN2, SLIT2, SNED1 and CCL2.

In a preferred embodiment said method is implemented taking into account that the soluble level of TSP1 and NID1 is higher, and the soluble level of CYR61 is lower, in subjects suffering from inflammatory angiogenesis as compared with the corresponding levels of said MT1-MMP substrates in subjects not suffering from inflammatory angiogenesis.

For the purpose of the present invention "soluble level" refers to the level of the MT1-MMP substrates found solubilised or dissolved in any biological fluid, for example plasma.

Moreover, the expression "higher level" refers to the soluble level of a given MT1-MMP substrate in a subject suffering from inflammatory angiogenesis wherein MT1-MMP is activated (MT1^{+/+}) which is greater than the corresponding soluble level of the same MT1-MMP substrate in a reference subject not suffering from inflammatory angiogenesis wherein MT1-MMP is not activated (MT1^{-/-}) (see **Figure 5B** for TSP1 and **Figure 5E** for NID1). Conversely, the expression "lower level" refers to the soluble level of a given MT1-MMP substrate in a subject suffering from inflammatory angiogenesis wherein MT1-MMP is activated (MT1^{+/+}) which is lower than the corresponding soluble level of the same MT1-MMP substrate in a reference subject not suffering from inflammatory angiogenesis wherein MT1-MMP is not activated (MT1^{-/-}) (see **Figure 6B** for CYR61).

The present invention also refers to an *in vitro* method for the diagnosis or prognosis of chronic inflammatory disorders that comprises the diagnosis or prognosis of inflammatory angiogenesis according to the above explained method. In a preferred embodiment, the chronic inflammatory disorder is selected from: psoriasis, rheumatoid arthritis or atherosclerosis.

It is important to note that the diseases psoriasis, rheumatoid arthritis and atherosclerosis are previously mentioned in the present invention as illustrative examples of disorders caused by inflammatory angiogenesis. However, it is not intended to limit the present invention to said three diseases because the MT1-MMP substrates indentified in the present invention can be used for the diagnosis or prognosis of several diseases, affecting different organs, related with an abnormal increase of the angiogenic response for example:
- Blood vessels: vascular malformations Di George syndrome, hereditary hemorrhagic telangiectasia, cavernous hemangioma, transplant arteriopathy, hypertension, diabetes and restenosis.
- Nervous system: multiple sclerosis, Alzheimer disease, amyotrophic lateral sclerosis, diabetic neuropathy and stroke.
- Numerous organs: cancer, infectious disease and autoimmune disorders.
- Bone, joints: synovitis, osteomyelitis, osteophyte formation, osteoporosis and impaired bone fracture healing.
- Skin: warts, allergic dermatitis, scar keloids, blistering disease (bullous pemphigoid, dermatitis herpetiformis, and erythema multiforme), Kaposi sarcoma, hair loss and skin purpura or telangiectasia.
- Gastrointestinal: inflammatory bowel and periodontal disease, ascites, peritoneal adhesions, chronic viral hepatitis, gastric or oral ulcerations and Crohn's disease.
- Lung: primary pulmonary hypertension, asthma, nasal polyps, neonatal respiratory distress, pulmonary fibrosis and emphysema.
- Eye: persistent hyperplasic vitreous syndrome, diabetic retinopathy, retinopathy of prematurity and choroidal neovascularization.
- Adipose tissue: obesity.
- Reproductive system: endometriosis, uterine bleeding, ovarian cysts, ovarian hyperstimulation, pre-eclampsia and menorrhagia.
- Kidney: nephropathy.

### BRIEF DESCRIPTION OF THE FIGURES

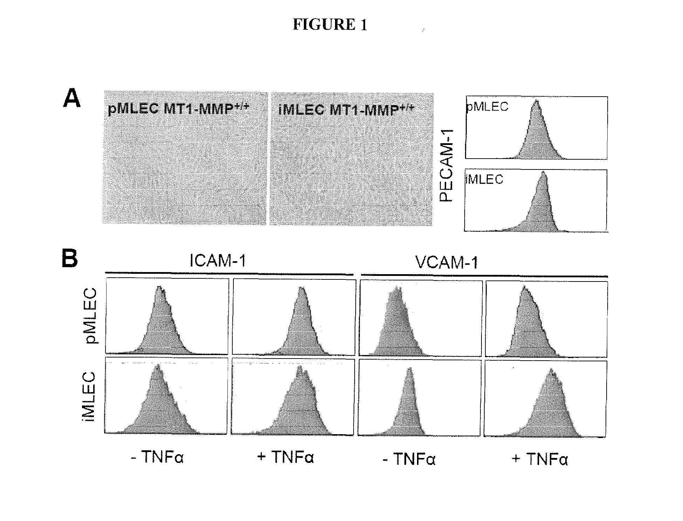

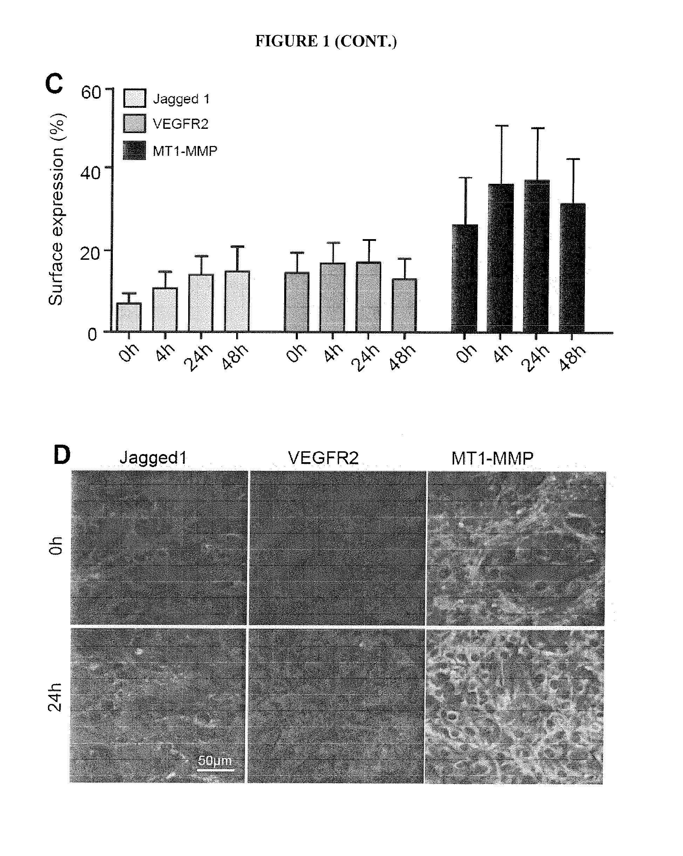

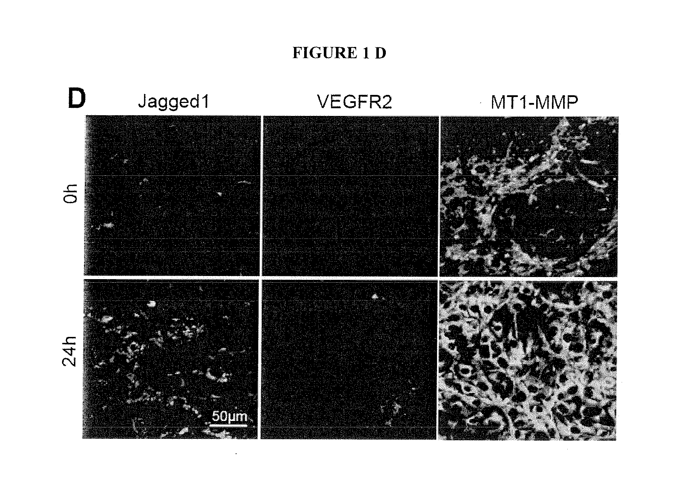

**Figure 1****.** TNFα-stimulated immortalized mouse lung endothelial cells (iMLEC) as a model of inflammatory endothelial tip cell priming. **A)** Phase contrast images showing morphology of primary MLEC (pMLEC) and iMLEC wildtype cultures. MLEC were immortalized by infection with polyoma virus middle T. Histograms show representative flow cytometry analysis of the cell-surface expression of the endothelial cell marker PECAM-1. **B)** Representative flow cytometry analysis of cell-surface expression of ICAM- and VCAM-1 in pMLEC and iMLEC stimulated with 20 ng/ml TNFα (n=3). C) Flow cytometry analysis of the cell-surface expression of the endothelial tip cell markers Jagged1 and VEGFR2 and MT1-MMP in TNFα-stimulated iMLEC. Bars represent percentage of expression (n=3). **D)** Confocal immunofuorescence staining of the same markers analyzed in C; representative maximal projection images are shown (n=3).

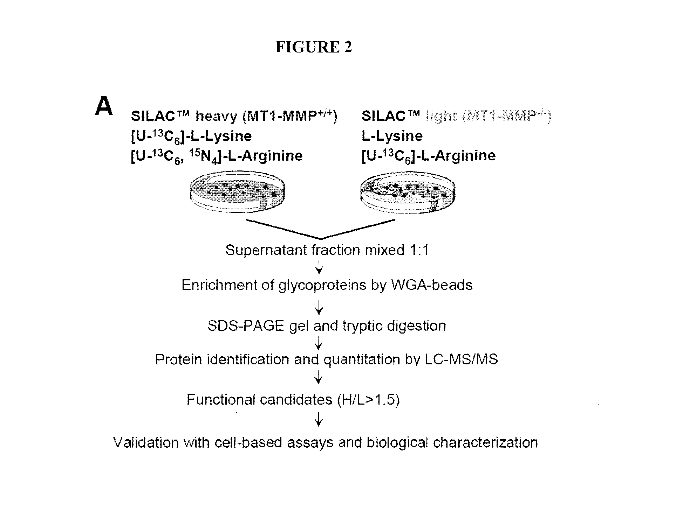

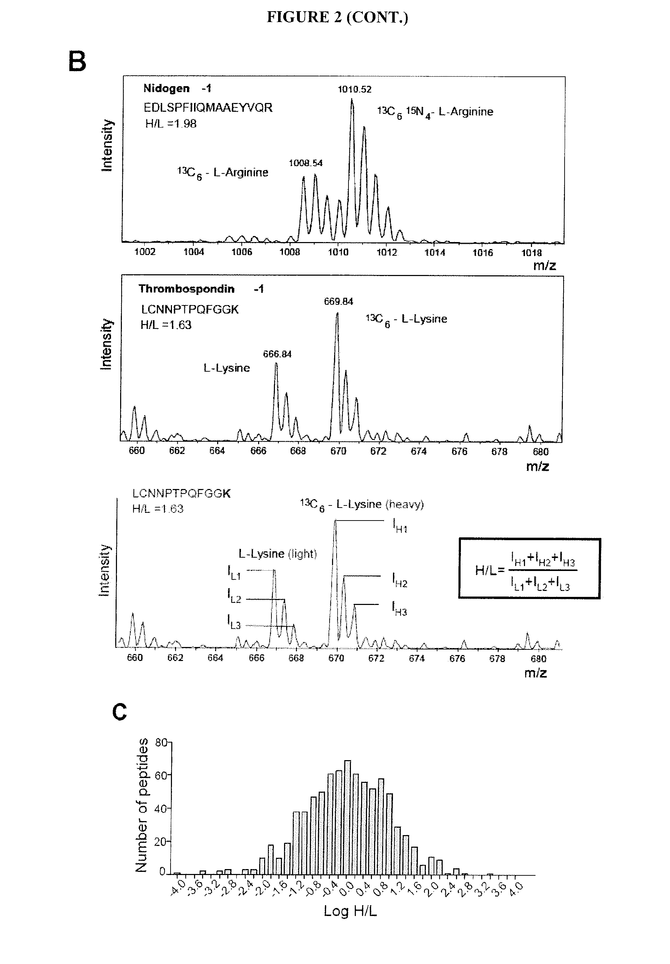

**Figure 2****.** SILAC comparison of wild type (WT) and MT1-MMP null iMLEC. **A)** SILAC strategy used to compare WT (MT1-MMP^{+/+}) and MT1-MMP null (MT1-MMP^{-/-}) iMLEC. **B)** Example peptide mass spectra for NID1 and TSP1, showing relative abundance of heavy- and light- labeled forms. C) Normal distribution of H/L ratios of labeled peptides.

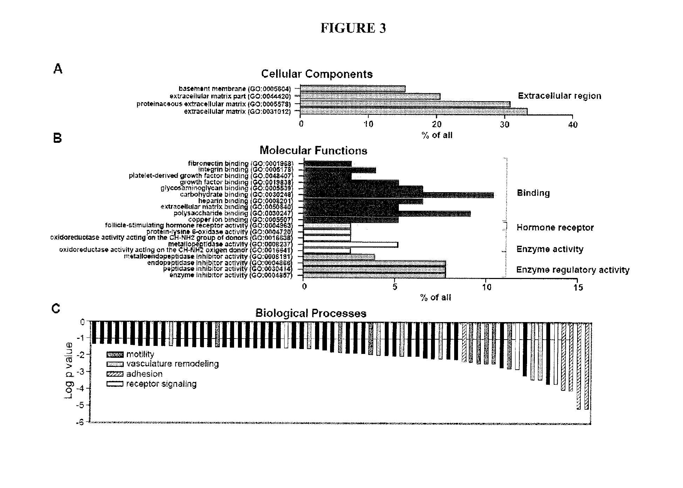

**Figure 3****.** Gene ontology (GO) terms analysis (Babelomics and FatiGO) of the MT1-MMP endothelial degradome. Identified MT1-MMP substrates with an H/L ratio >1.5 were analyzed for GO terms. **A)** and **B)** Percentages of total identified GO Cellular Components and Molecular Functions terms. **C)** Logarithmic p-values for enrichment in GO Biological Processes terms.
**Figure 4****.** Systems biology analysis reveals a combinatorial MT1-MMP proteolytic program during angiogenesis. Modules group substrates according to four general GO Biological Processes terms relevant to angiogenesis: adhesion, motility, chemotaxis and vessel development. Continuous lines demark the general GO Biological Processes and dotted lines demark more restricted processes within these. Proteins that appear to be exclusive MT1-MMP substrates within the metalloproteinase family are underlined. Proteins encoded by genes upregulated in inflammatory-activated wildtype pMLEC are highlighted in gray.
**Figure 5****.** Validation of TSP1 and NID1 processing by MT1-MMP in primary endothelial cells. **A)** Structure of TSP1. Arrows mark potential MT1-MMP cleavage sites (only those beginning with R) and gray-shaded areas indicate SILAC-identified tryptic peptides; examples of H/L ratios >1.5 are indicated. The domain structure of TSP1 is also shown. **B)** Immunoblot detection of TSP1 in total lysates (TL) and culture supernatants (CSUP) of TNFα-stimulated WT (MT1^{+/+}) and null (MT1^{-/-}) pMLEC. The bar chart shows densitometric quantification of TSP1 in supernatants (n=3). C) Confocal immunofluorescence of TSP1 and F-actin (in insets) in TNFα-stimulated WT (MT1-MMP^{+/+}) and null (MT1-MMP^{-/-}) pMLEC; representative maximal projections are shown (n=5). **D)** Structure of NID1. Arrows mark potential MT1-MMP cleavage sites (only those beginning with R) and gray-shaded areas indicate SILAC-identified tryptic peptides; examples of H/L ratios >1.5 are indicated. The domain structure of NID1 is also shown. **E)** Immunoblot detection of NID1 in total lysates (TL) and supernatants (CSUP) of TNFα-stimulated WT (MT1^{+/+}) and null (MT1^{-/-}) pMLEC. The bar chart shows densitometric quantification of NID1 in supernatants (n=3). F) Confocal immunofluorescence of NID1 and F-actin (in insets) in TNFα-stimulated WT (MT1-MMP^{+/+}) and null (MT1-MMP^{-/-}) pMLEC; representative maximal projections are shown (n=5).

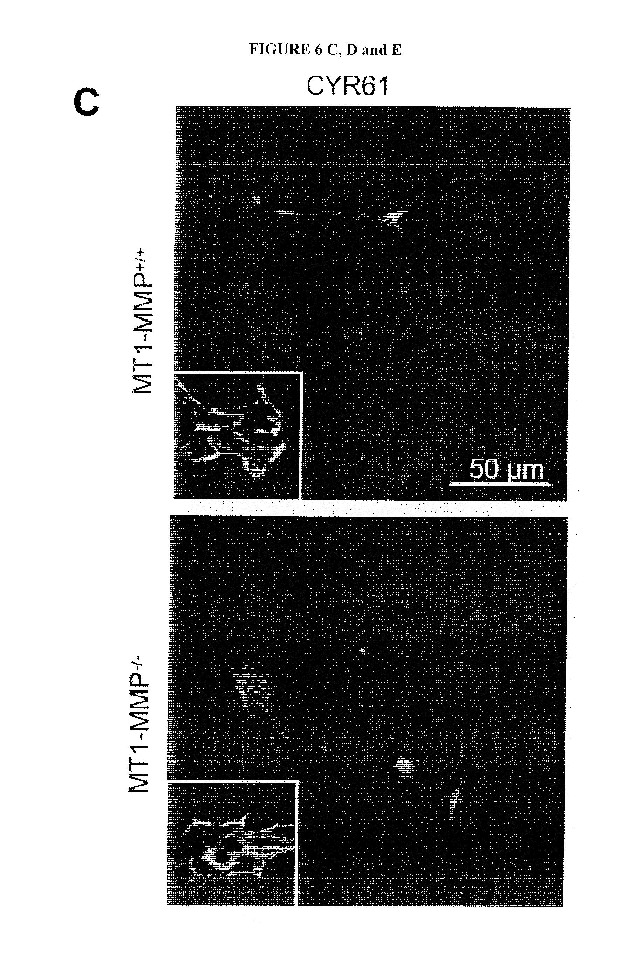

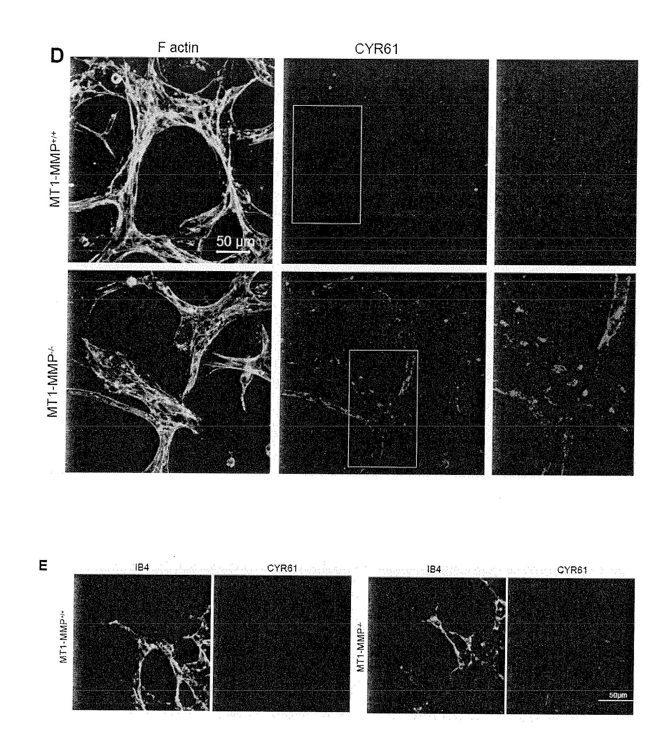

**Figure 6****.** CYR61 processing is impaired in MT1-MMP null endothelial tip cells *in vitro* and *in vivo.* **A)** Structure of CYR61. Arrows mark potential MT1-MMP cleavage sites (only those beginning with R) and gray-shaded areas indicate SILAC-identified tryptic peptides. H/L ratios are boxed for H/L >1.5 and for H/L <1; the representative gel shows corresponding migration positions on SDS-PAGE in the SILAC procedure. The domain structure of CYR61 is also shown. **B)** Immunoblot detection of CYR61 in CSUP and TL of TNFα-stimulated WT (MT1^{+/+}) and null (MT1^{-/-}) pMLEC. The bar chart shows densitometric quantification of CYR61 levels in supernatants (n=3). C) Confocal immunofluorescence of CYR61 and F-actin in TNFα-stimulated WT (MT1-MMP^{+/+}) and null (MT1-MMP^{-/-}) pMLEC; representative maximal projections and a magnified detail are shown (n=5). **D)** Confocal immunofluorescence as in C in TNFα-stimulated WT and null pMLEC grown in Ibidi chambers covered by 3D-Matrigel; representative maximal projections are shown (n=5). **E)** Whole mount confocal immunofluorescence of isolectin B4 and CYR61 in retinas of P6 WT (MT1-MMP^{+/+}) and null (MT1-MMP^{-/-}) mice; representative maximal projections are shown (n=4).
**Figure 7****.** MT1-MMP proteolytic program in inflammatory angiogenesis. Combined processing of at least the MT1-MMP substrates CYR61, NID1 and TSP1 has a major impact on the regulation of endothelial cell adhesion, migration, chemotaxis, and sprouting, ultimately leading to vessel morphogenesis. This proteolytic program is activated by inflammatory stimuli such as TNFα. The absence of MT1-MMP in stimulated endothelial cells results in impaired processing and accumulation of the identified substrates and defective angiogenesis.
**Figure 8****.** Soluble levels of the MT1-MMP identified substrates TSP1, NID1 and CYR61 were analyzed in supernatants of cultured endothelial cells obtained from 5 mouse littermates (3 KO and 2 WT). Single comparison of TSP1 soluble levels between for instance WT1 and KO1 would not yield a conclusive difference (accordingly to non-significant average difference in soluble TSP1 in Figure 5B). However, if ratio of soluble levels of NID1 and CYR61 from the same individuals is also calculated, in combination with TSP1, evident differences are found indicating that the combined assessment of these three soluble markers increase sensitivity and decrease false negative results due to inter-individual variations. It is important to note the higher levels of TSP1 and NID1, and the lower level of CYR61, in WT expressing MT1-MMP, as compared with KO not expressing MT1-MMP

### EXAMPLES

### Example 1. TNFα-activated immortalized mouse lung endothelial cells mimic endothelial tip cells.

Mouse lung endothelial cells (MLEC) are a useful model for investigating the contribution of MT1-MMP to inflammation-induced capillary formation (*Genis L et al 2007, Galvez BG et al 2005).* However, primary MLEC (pMLEC) only undergo a few passages and the short lifespan (p10) of MT1-MMP null mice limits the numbers of cells obtained. In order to ensure sufficient numbers of cells, primary MLEC from WT or MT1-MMP null mice were immortalized by infection with polyoma virus middle T, which activates cell growth and proliferation via stimulation of Akt and MAPK cascades (*Kobayashi H et al 2010*). Immortalized MLEC (iMLEC) maintained endothelial cell morphology and expression of the endothelial cell marker PECAM-1 (**Figure 1A**). iMLEC also expressed ICAM-1 and VCAM-1 normally in response to TNFα treatment and were able to form capillary tubes (**Figure 1B**). TNFα also induced expression of the endothelial tip cell markers Jagged1 and VEGFR2, as assessed by flow cytometry and immunostaining. MT1-MMP was also abundantly expressed under these conditions (**Figure 1C** and **1D**).

### Example 2. Use of SILAC approach for characterizing MT1-MMP substrates (degradome) in TNFα-stimulated endothelial cells.

Once the model for inflammation-driven endothelial tip cell priming was established (see **Example 1**), a further step was carried out in the present invention in order to define the MT1-MMP degradome by SILAC. Wildtype iMLEC were labeled with essential amino acids containing heavy "H" non-radioactive isotopes (¹³C₆ lysine and ¹³C₆ ¹⁵N₄ arginine), whereas MT1-MMP null iMLEC were labeled with light "L" isotopes (lysine and the ¹³C₆ form of arginine).

Details of the SILAC procedure are given in the scheme of **Figure 2A****.** MLEC were grown in complete SILAC Advance D-MEM F-12 Flex Medium (Invitrogen, Belgium) supplemented with 'light' and 'heavy' amino acids and dialyzed serum to exclude the possibility of incorporating non-labeled amino acids. Non-labeled lysine and the ¹³C₆ form of arginine (light "L" conditions by culturing the cells in the presence of forms of the amino acids (isotopes) with lower molecular mass) were used for MT1-MMP null MLEC, and isotopes of lysine (¹³C₆) and arginine (¹³C₆ ¹⁵N₄) for WT MLEC (heavy "H" conditions by culturing the cells in the presence of forms of the amino acids (isotopes) with higher molecular mass). Cells were cultured for 7 days, corresponding to about 6 doublings, and appropriate amounts of isotopic lysine and arginine were used to optimize isotopic incorporation while minimizing the risk of arginine to proline conversion. For the last 48 hours of culture, the medium was replaced by serum-free medium, to reduce the serum protein background, and for the last 24 hours cells were stimulated with 20 ng/ml TNFα (PeproTech, USA).

At the end of the 7 day period, conditioned medium from null and WT cultures was collected and the cells harvested with cold PBS + 10 mM EDTA. Supernatants from null ('light' condition) and wildtype (WT) ('heavy' condition) were pooled 1:1 after bicinchoninic acid protein determination (BCA Protein Assay, Pierce, USA). The pooled media were incubated for 3 hours at 4°C with WGA-agarose beads to extract glycoproteins, and the glycoproteins were eluted by incubation with 0.5 M N-acetyl-D-glucosamine. The glycoprotein mixture was concentrated on a 3 kDa cut-off centrifugal filter (Amicon Ultra-4, Millipore, Ireland) to a final volume of 200 µl. Urea was added to a final concentration of 8M, pH 8.5. Proteins were reduced by addition of 50 mM DDT, followed by carbamidomethylation with 125 mM iodoacetamide. Finally, the protein mixture was purified by acetone-trichloroacetic acid precipitation (2D-CleanUp kit, GE Healthcare). The pellet was resuspended in loading buffer, and proteins were separated by 10% SDS-PAGE.

After separation of glycoproteins by 10% SDS-PAGE, Coomassie-stained gel was cut into 20 vertically distributed slices, and each one was in-gel digested with modified porcine trypsin (Promega, USA). The digests were dried in a vacuum centrifuge, extracted with formic acid solution, and analyzed on an Esquire Ultra IT mass spectrometer (Bruker, Bremen, Germany) coupled to a nano-HPLC system (Ultimate, LC Packings, The Netherlands). Peptide mixtures were first concentrated on a 300 mm i.d. (inner diameter), 1 mm PepMap nanotrapping column and then loaded onto a 75 mm i.d., 15 cm PepMap nanoseparation column (LC Packings, The Netherlands). Peptides were then eluted with an acetonitrile (ACN) gradient (0-60% B in 150 min, where B is 80% ACN, 0.1% formic acid in water; flow rate ca. 300 nl/min) through a PicoTip emitter nanospray needle (New-Objective, Woburn, MA, USA) onto the nanospray ionization source of the IT mass spectrometer. MS/MS fragmentation (1.9 s, 100-2800 m/z) was performed on three of the most intense ions, as determined from a 1.2 s MS survey scan (310-1500 m/z), using a dynamic exclusion time of 1.2 min for precursor selection and excluding single-charged ions.

Data were processed for protein identification and quantification with Protein Scape 2.1 and WARP-LC 1.2 (Bruker, Bremen, Germany), a software platform that integrates processing of LC-MS run data, protein identification through database search of MS/MS spectra, and protein quantification based on the integration of the chromatographic peaks of MS extracted ion chromatograms for each precursor. Proteins were identified using Mascot (Matrix Science, London, UK) to search the SwissProt database, restricting the search to mouse proteins. MS/MS spectra were searched with a precursor mass tolerance of 1.5 Da, fragment tolerance of 0.5 Da, trypsin specificity with a maximum of 1 missed cleavage, cysteine carbamidomethylation as a fixed modification, and methionine oxidation and the N-terminal and corresponding Lys and Arg SILAC labels as variable modifications. A positive identification criterion was set as an individual Mascot score for each peptide MS/MS spectrum higher than the corresponding homology threshold score. The false positive rate for Mascot protein identification was measured by searching a randomized decoy database as described (*Elias JE et al 2007*) and estimated to be under 4%. For protein quantification, H/L ratios were calculated by averaging the measured H/L ratio for the observed peptides, after discarding outliers. For selected proteins of interest, quantitative data obtained from the automated Protein Scape software analysis were manually reviewed. Further protein analysis was conducted with Protein Knowledgebase UniProtKB (http://www.uniprot.org) and GeneCards database (http://www.genecards.org).

After approximately six doublings, supernatant glycoproteins from WT and MT1-MMP null cultures were mixed and separated by SDS-PAGE, and trypsin-digested gel slices were analyzed by mass spectrometry. More than 800 peptides with well-defined mass spectra were used to quantify protein ratios between WT (heavy, H) and null (light, L) iMLEC supernatants. Examples of mass spectra are shown in **Figure 2****B.** As expected, most proteins were unaffected and therefore the frequency of ratios fits the Gaussian distribution with a peak at Log H/L = 0 (**Figure 2C**). Interpretation of the ratio for a given protein depends on protein type. For example, a transmembrane protein shed by MT1-MMP will be more frequent in conditioned medium of WT cells, yielding an H/L ratio >1. On the other hand, a secreted protein degraded by MT1-MMP might be more frequent in medium of null cells, giving an H/L ratio <1 (*Doucet A et al 2008).*

Therefore a complete list of proteins were identified and quantified according to the relative H/L ratio in the supernatants of TNFα-activated iMLEC. Functional categorization of the identified proteins, based on their generic assigned function, defined a group of angiogenesis-related proteins (**Table 1**). Although most of the identified proteins were glycoproteins, a small number of intracellular proteins were also detected. This might be related to non-specific release of cell proteins by apoptotic cells or might reflect alternative functions of these processed intracellular proteins in the extracellular milieu.

### Example 3. MT1-MMP endothelial degradome: non-exclusive and exclusive substrates.

The definition of the MT1-MMP endothelial degradome was limited, in the present invention, to extracellular glycoproteins with an H/L ratio >1.5 to focus on those substrates with more probability to be shed by MT1-MMP from the cell membrane or from the extracellular matrix. This threshold was selected on the basis of the ratio for the established MT1-MMP substrate collagen type III (1.67). **Table 1** comprises both non-exclusive and exclusive substrates of MT1-MMP. Among these non-exclusive substrates were several extracellular matrix (ECM) proteins such as TSP1 and TSP2, collagen III and VI, fibronectin, fibrillin, laminin, tenascin, and nidogen. Exclusive MT1-MMP substrates included ECM components such as CYR61, fibulin and SNED1, and non-ECM proteins such as PR2C2, SLIT, EGFR and SFRP1. This profile includes proteins involved in angiogenesis, and might reflect the essential role of MT1-MMP in this process.

### Example 4. MT1-MMP activity modulates key biological processes in TNFα-activated endothelial cells.

The Cellular Components Gene Ontology (GO) (*Harris MA et al 2004*) terms for the identified MT1-MMP substrates were characterized in order to assess their subcellular location and likely exposure to the protease. The gene set was analyzed with the FatiGO (*Al-Shahrour F et al 2004*) module in the open-source bioinformatics web tool Babelomics (*Medina I et al 2010*), which compares genes of interest with the rest of the mouse genome. The Babelomics web tool (http//www.babelomics.org/) is a freely available program suited to analysis of large-scale experiments (microarrays, proteomics) with functional annotation (*Medina I et al 2010*). The FatiGO *(Al-Shahrour F et al 2004)* module was used for enrichment analysis of selected proteins. This functional profiling was performed by comparing two lists of genes, the first being the genes encoding the proteins with an H/L ratio > 1.5 in the SILAC analysis unless otherwise stated, and the second being the rest of the genome. The FatiGO analysis indicated that potential MT1-MMP substrates are preferentially located in extracellular and matrix-related compartments (**Figure 3A****)**. This was supported by FatiGO analysis of Molecular Functions GO terms, which revealed enrichment of matrix and matrix-binding related functions in the MT1-MMP endothelial degradome (**Figure 3B**). GO analysis of Biological Processes showed an enrichment of the MT1-MMP degradome in processes related to cellular adhesion and motility, key steps in endothelial tip cell function, and to vascular remodeling (**Figure 3C**). Some of the same biological processes were also enriched in the set of proteins with an H/L ratio <1.5, suggesting that this set might also include MT1-MMP substrates relevant to the angiogenic response.

### Example 5. The MT1-MMP degradome suggests a combinatorial proteolytic program during angiogenesis.

The functional clustering of selected MT1-MMP substrates included in **Table 1** suggests a combinatorial model of processing during inflammatory activation of endothelial cells. In this model MT1-MMP substrates are grouped into specific functional modules linked to GO Biological Processes related to angiogenesis (see **Figure 4**). Most of these substrates are exclusive to MT1-MMP within the metalloproteinase family, with TSP1, NID1, CO3A1 and SEM3C being the main exceptions (**Table 1**). TSP1 seems to be critical for the MT1-MMP response since it is present in most of the functional modules. TSP1 can also be processed by ADAMTS1, which is upregulated in the transcriptome of WT pMLEC activated with the inflammatory chemokine MCP-1, suggesting that these two proteases and TSP1 cooperate during angiogenesis. Other identified substrates such as MMP19, CO3A1 and LYOX are also upregulated in the transcriptome of MCP-1-activated WT MLEC, suggesting that their relative abundance in the supernatant of WT MLEC might be related to regulation of their transcription or processing by MT1-MMP

Processing of TSP1 in combination with MT1-MMP-mediated cleavage of other substrates would lead, preferentially, to specific downstream outcomes. For example, co-processing with NID1 would affect endothelial cell adhesion, while processing with PR2C2 or SLIT2 would affect motility, and processing with CYR61 would preferentially affect chemotaxis. Additional processing of other substrates in the modules, such as SNED1, FBLN2, EGFR, CSF1, CCL2 or SEM3C, would further modulate these biological processes, leading finally to angiogenesis and vessel morphogenesis (**Figure 4**). Interestingly, some of these proteins, such as SNED1, were not previously identified as related to angiogenesis (**Table 1**). In the proposed model, modules can be cross-combined: while the processing of some MT1-MMP substrates is linked to defined functions (such as CCL2 or SLIT2 to cell motility and chemotaxis), the processing of substrates with broader roles will affect multiple functions. For example, CYR61 could contribute to cell adhesion, chemotaxis or vascular development depending on the accompanying substrates (**Figure 4**). This system biology analysis thus indicates the existence of finely-tuned and orchestrated rather than random proteolysis during the angiogenic response.

### Example 6. Processing of TSP1 and NID1 is altered in MT1-MMP null endothelial cells.

TSP1 and NID1 are key components of the combinatorial MT1-MMP proteolytic program proposed in the present invention. To confirm these proteins as MT1-MMP substrates, TSP1 and NID1 sequences were analyzed for the presence of the preferential MT1-MMP cleavage sequence R(F/K)-nonP-X(polar)-X(hydrophobic) *(Kridel SJ et al 2002).* Both TSP1 and NID1 contain several putative MT1-MMP cleavage sequences at distinct positions. Moreover, some of them predict peptides corresponding to fragments identified in the SILAC analysis, suggesting possible proteolysis at these sites (**Figure 5A** and **5D**).

To validate the SILAC and in silico data experimentally, TSP1 and NID1 processing was analyzed in pMLEC treated with TNFα for 24 h. TSP1 levels were similar in total cell lysates from WT and MT1-MMP null MLEC, but levels of soluble TSP1 were higher in culture supernatants of WT MLEC, indicating release of TSP1 from the matrix by MT1-MMP (**Figure 5B** and **Figure 7**). Accordingly, MT1-MMP-deficient MLEC accumulated larger TSP1 pericellular clusters compared with WT MLEC (**Figure 5C**).

Although NID1 was undetectable in total lysates, levels of soluble NID1 were also significantly increased in culture supernatants from TNFα-stimulated WT pMLEC, suggesting MT1-MMP-dependent release of NID1 from the basement membrane (**Figure 5E**). Consistent with cleavage of NID1 at the N- or C-terminus, the molecular weight of NID1 in supernatants from WT MLEC was slightly smaller (**Figure 5E**).

Accordingly, MT1-MMP-deficient MLEC, but not their WT counterparts, accumulated NID1 at clusters in contact with the matrix (**Figure 5F** and **Figure 7**).

### Example 7. Impaired CYR61 processing results in its accumulation in MT1-MMP null endothelial cells in vitro and in vivo.

Matricellular proteins are extracellular proteins that bind to and regulate matrix proteins and cell surface receptors but are not themselves structural ECM elements. The matricellular protein CYR61 (also known as CCN1) is implicated in vascular development. In the combinatorial model of the present invention (**Figure 4**), proteolysis of CYR61 is a key regulatory step since its processing in combination with distinct substrates, preferably with TSP1 and NID1, can impact different biological processes required for angiogenesis. To investigate CYR61 processing by MT1-MMP in angiogenesis, it was first confirmed the presence of several MT1-MMP cleavable sequences in CYR61 (**Figure 6A**). CYR61 levels in total lysates were unaffected by MT1-MMP deficiency, but, in contrast to TSP1 and NID1, levels of soluble CYR61 in supernatants of TNFα-activated WT MLEC were lower than in supernatants of similarly treated null cells (**Figure 6B**).

This unexpected finding might be explained by the relative electrophoretic positions of CYR61 proteolytic products and the associated H/L ratios. Two peptides were quantified by SILAC with H/L ratios >1.5, confirming CYR61 as potential MT1-MMP substrate. These peptides derive from the digestion of gel-slice 20 (lowest MW on **Figure 6**), which probably corresponds to a small processed fragment of CYR61 undetectable by the antibody used for western blot. In contrast, two other peptides yielded H/L ratios <0.5 on primary analysis, indicating higher abundance in null cells. These peptides derive from gel-slices 15-16 (-40 kDa), the expected MW of intact CYR61, thus explaining the increased levels of intact CYR61 observed in **Figure 6****B.** This finding indicates that CYR61 is normally processed by MT1-MMP and that the soluble unprocessed form therefore accumulates in null cells. This is supported by an enrichment of CYR61 at adhesion sites in null MLEC, compared with a fainter staining in WT cells (**Figure 6C**).

To better define the function of MT1-MMP-mediated CYR61 processing in endothelial tip cells and vessel formation, the distribution of CYR61 in 3D-Matrigel MLEC cultures was analyzed. Matrigel (BD Biosciences, USA) was thawed overnight on ice at 4°C and all materials were chilled before use. MLEC were plated at confluence in ibidi® angiogenic chambers and left to attach, after which the Matrigel layer was added on top. Medium containing TNFα was added and the chamber was kept at 37 °C for at least 18 h. At the end of incubation, medium was removed and the cells were fixed with freshly prepared 4% PFA for 20 min, followed with permeabilization in 0.3% Triton X-100. Samples were washed with PBS/Triton X-100 and stained for CYR61. Samples were examined under a Zeiss LSM700 confocal microscope (Plan-Apochromat 40x1.3 Oil DIC M27) and images were analyzed with ZEN software.

An enhanced CYR61 signal was observed in pericellular clusters in MT1-MMP null endothelial tip cells (**Figure 6D**). To investigate the distribution of CYR61 in a physiological setting, retinal vascularization in postnatal WT and MT1-MMP null mice was examined, since this tissue is characterized by active angiogenesis and matrix remodeling at this stage. Therefore, eyes from 6 to 8 days-old mice (P6-P8) were removed and fixed with 4% PFA overnight at 4°C. Fixed retinas were flat mounted and blocked in 2% BSA/PBS/0.3% Triton X-100 followed by overnight incubation with primary antibodies (anti-CYR61) and isolectin IB4 (Sigma) to visualize vasculature. The next day retinas were extensively washed and incubated with fluorescent-conjugated secondary antibodies. Images were acquired with a Zeiss LSM700 confocal microscope (Plan-Apochromat 63x1.4 Oil DIC M27) and images were analyzed with ZEN software.

Accumulation of CYR61 was detected around endothelial tip cells at the vascular front of retinas from MT1-MMP null neonates but not their WT counterparts, indicating that this newly-identified MT1-MMP processing axis is relevant to *in vivo* angiogenesis (**Figure 6E**).

### Example 8. Combination of TSP1, NID1 and CYR61 as biomarkers.

This example shows that endothelial cells are activated and express active MT1-MMP in chronic inflammatory disease and, conversely, that, in non-inflammatory normal conditions, MT1-MMP is barely or not expressed in endothelial cells.

Soluble levels of the MT1-MMP identified substrates TSP1, NID1 and CYR61 were analyzed in supernatants of cultured endothelial cells obtained from 5 mouse littermates (3 KO and 2 WT). Single comparison of TSP1 soluble levels between for instance WT1 and KO1 would not yield a conclusive difference (accordingly to non-significant average difference in soluble TSP1 in Figure 5B). However, if ratio of soluble levels of NID1 and CYR61 from the same individuals is also calculated, in combination with TSP1, evident differences are found indicating that the combined assessment of these three soluble markers increase sensitivity and decrease false negative results due to inter-individual variations. It is important to note the higher levels of TSP1 and NID1, and the lower level of CYR61, in WT expressing MT1-MMP, as compared with KO not expressing MT1-MMP (see **Figure 8**).

### REFERENCES

1. Genis L, Gonzalo P, Tutor AS, et al. Functional interplay between endothelial nitric oxide synthase and membrane type 1 matrix metalloproteinase in migrating endothelial cells. Blood. 2007;110:2916-2923.
2. Galvez BG, Genis L, Matias-Roman S, et al. Membrane type 1-matrix metalloproteinase is regulated by chemokines monocyte-chemoattractant protein-1/ccl2 and interleukin-8/CXCL8 in endothelial cells during angiogenesis. J Biol Chem. 2005;280:1292-1298.
3. Doucet A, Butler GS, Rodriguez D, Prudova A, Overall CM. Metadegradomics: toward in vivo quantitative degradomics of proteolytic post-translational modifications of the cancer proteome. Mol Cell Proteomics. 2008;7:1925-1951.
4. Overall CM, Dean RA. Degradomics: systems biology of the protease web. Pleiotropic roles of MMPs in cancer. Cancer Metastasis Rev. 2006;25:69-75.
5. Ong SE, Blagoev B, Kratchmarova I, et al. Stable isotope labeling by amino acids in cell culture, SILAC, as a simple and accurate approach to expression proteomics. Mol Cell Proteomics. 2002;1:376-386.
6. Elias JE, Gygi SP. Target-decoy search strategy for increased confidence in large-scale protein identifications by mass spectrometry. Nat Methods. 2007;4:207-214.
7. Medina I, Carbonell J, Pulido L, et al. Babelomics: an integrative platform for the analysis of transcriptomics, proteomics and genomic data with advanced functional profiling. Nucleic Acids Res. 2010;38:W210-213.
8. Al-Shahrour F, Diaz-Uriarte R, Dopazo J. FatiGO: a web tool for finding significant associations of Gene Ontology terms with groups of genes. Bioinformatics. 2004;20:578-580.
9. Kobayashi H, Butler JM, O'Donnell R, et al. Angiocrine factors from Akt-activated endothelial cells balance self-renewal and differentiation of haematopoietic stem cells. Nat Cell Biol. 2010;12:1046-1056.
10. Harris MA, Clark J, Ireland A, et al. The Gene Ontology (GO) database and informatics resource. Nucleic Acids Res. 2004;32:D258-261.
11. Kridel SJ, Sawai H, Ratnikov BI, et al. A unique substrate binding mode discriminates membrane type-1 matrix metalloproteinase from other matrix metalloproteinases. J Biol Chem. 2002;277:23788-23793.

## Claims

1. The group of MT1-MMP substrates comprising: TSP1, NID1 and CYR61 for use as biomarker of inflammatory angiogenesis.

2. The group of MT1-MMP substrates comprising: TSP1, NID1 and CYR61, according to claim 1, for use as biomarker of inflammatory angiogenesis in combination with at least one of the following MT1-MMP substrates: EGFR, PR2C2, SEM3C, CSF1, FBLN2, SLIT2, SNED1 or CCL2, or combinations thereof.

3. The group of MT1-MMP substrates: TSP1, NID1, CYR61, EGFR, PR2C2, SEM3C, CSF1, FBLN2, SLIT2, SNED1 and CCL2, according to claim 2, for use as biomarker of inflammatory angiogenesis.

4. The group of MT1-MMP substrates comprising: TSP1, NID1 and CYR61 for use as biomarker for the *in vitro* diagnosis or prognosis of disorders caused by inflammatory angiogenesis.

5. The group of MT1-MMP substrates: TSP1, NID1 and CYR61, according to claim 4, for use as biomarker for the *in vitro* diagnosis or prognosis of disorders caused by inflammatory angiogenesis, in combination with at least one of the following MT1-MMP substrates: EGFR, PR2C2, SEM3C, CSF1, FBLN2, SLIT2, SNED1 or CCL2, or combinations thereof.

6. The group of MT1-MMP substrates: TSP1, NID1, CYR61, EGFR, PR2C2, SEM3C, CSF1, FBLN2, SLIT2, SNED1 and CCL2, according to claim 5, for use as biomarker for the *in vitro* diagnosis or prognosis of disorders caused by inflammatory angiogenesis.

7. The group of MT1-MMP substrates, according to claims 4 to 6, for use as biomarker for the *in vitro* diagnosis or prognosis of chronic inflammatory disorders selected from: psoriasis, rheumatoid arthritis or atherosclerosis.

8. *In vitro* method for the diagnosis or prognosis of inflammatory angiogenesis which comprises assessing the soluble level of at least the following group of MT1-MMP substrates: TSP1, NID1 and CYR61, in samples isolated from the subject under investigation.

9. *In vitro* method, according to claim 8, further comprising the assessment of the soluble level of at least one of the following MT1-MMP substrates: EGFR, PR2C2, SEM3C, CSF1, FBLN2, SLIT2, SNED1 or CCL2, or combinations thereof.

10. *In vitro* method, according to claim 9, comprising the assessment of the soluble level of the following group of MT1-MMP substrates: TSP1, NID1, CYR61, EGFR, PR2C2, SEM3C, CSF1, FBLN2, SLIT2, SNED1 and CCL2.

11. *In vitro* method, according to claim 8, wherein the soluble level of TSP1 and NID1 is higher, and the soluble level of CYR61 is lower, in subjects suffering from inflammatory angiogenesis as compared with the corresponding levels of said MT1-MMP substrates in subjects not suffering from inflammatory angiogenesis.

12. *In vitro* method for the diagnosis or prognosis of chronic inflammatory disorders that comprises the diagnosis or prognosis of inflammatory angiogenesis according to the method of claims 8 to 11.

13. *In vitro* method, according to claim 12, wherein the chronic inflammatory disorders is selected from: psoriasis, rheumatoid arthritis or atherosclerosis.
